# EUROPEAN PATENT SPECIFICATION

(11) **EP 1 781 651 B1**
(45) Date of publication and mention of the grant of the patent: **11.03.2009**
(21) Application number: 05762590.7
(22) Date of filing: 08.07.2005
(51) Int. Cl.: C07D 453/02

(54) **PROCESS FOR PREPARING QUINUCLIDINIUM CARBAMATE DERIVATIVES**
VERFAHREN ZUR HERSTELLUNG VON CHINUCLIDINIUMCARBAMATDERIVATEN
PROCEDE POUR PREPARER DES DERIVES DE CARBAMATE DE QUINUCLIDINIUM

(30) Priority: 29.07.2004 ES 200401880
(43) Date of publication of application: 09.05.2007
(73) Proprietor: Laboratorios Almirall, S.A., 08022 Barcelona (ES)
(72) Inventor: PRAT QUINONES, Maria, E-08003 Barcelona (ES); BUSQUETS BAQUE, Nuria, E-08024 Barcelona (ES); PUJOL NOGUERA, Ferran, E-08635 Sant Esteve de Sesrovires (Barcelona) (ES); IBARZO CASAMIAN, Francisco, Javier, E-08202 Sabadell (Barcelona) (ES)
(74) Representative: Winkler, Andreas Fritz Ernst
(86) International application number: PCT/EP2005/007424
(87) International publication number: WO 2006/010452

(56) References cited:
- WO-A-03/053966
- WO-A-20/04000840
- REINER ET AL.: "3-Hydroxyquinucldinium derivatives: synthesis of compounds and inhibition of acetylcholinesterase" CHEMO-BIOLOGICAL INTERACTIONS, vol. 119-120, 1999, pages 173-181, XP002320141

## Description

The present invention relates to a new process for preparing carbamate derivatives having the structure of formula (I): 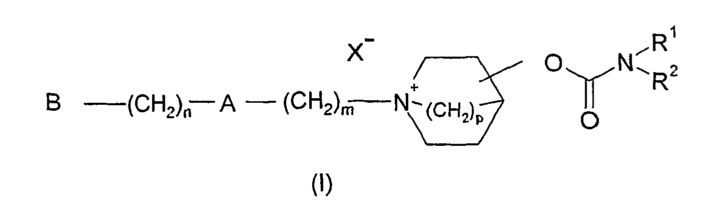 wherein:
R¹ represents a group selected from phenyl, 2-furyl, 3-furyl, 2-thienyl, 3-thienyl, benzyl, furan-2-ylmethyl, furan-3-ylmethyl, thiophen-2-ylmethyl, thiophen-3-ylmethyl;
R² represents a group selected from optionally substituted lower alkyl, optionally substituted lower alkenyl, optionally substituted lower alkynyl, saturated or unsaturated cycloalkyl, saturated or unsaturated cycloalkylmethyl, phenyl, benzyl, phenethyl, furan-2-ylmethyl, furan-3-ylmethyl, thiophen-2-ylmethyl, thiophen-3-ylmethyl, pyridyl, and pyridylmethyl; wherein the carbocyclic moieties in the cycloalkyl, cycloalkylmethyl, phenyl, benzyl or phenethyl groups can be optionally bridged or fused to another saturated, unsaturated or aromatic carbocyclic moiety or to a cyclic moiety comprising carbon atoms and 1 or 2 oxygen atoms;
   the cyclic groups present in R¹ and R² being optionally substituted by one, two or three substituents selected from halogen, straight or branched, optionally substituted lower alkyl, hydroxy, straight or branched, optionally substituted lower alkoxy, -SH, straight or branched optionally substituted lower alkylthio, nitro, cyano, -NR'R", -CO₂R', -C(O)-NR'R", -N(R"')C(O)-R', -N(R"')-C(O)NR'R", wherein R', R" and R"' each independently represents a hydrogen atom or a straight or branched, optionally substituted lower alkyl group or R' and R" together with the atom to which they are attached form a cyclic group;
p is 1 or 2 and the carbamate group is attached at positions 2, 3 or 4 of the azoniabicyclic ring,
m is an integer from 0 to 8;
n is an integer from 0 to 4;
A represents a group selected from -CH₂-, -CH=CR'-, -CR'=CH-, -CR'R"-, -C(O)-, -O-, -S-, -S(O)-, -S(O)₂- and -NR'-, wherein R' and R" are as defined above;
B represents a hydrogen atom, or a group selected from straight or branched, optionally substituted lower alkyl, hydroxy, straight or branched, optionally substituted lower alkoxy, cyano, nitro, -CH=CR'R", -C(O)OR', -OC(O)R', -SC(O)R', -C(O)NR'R", -NR'C(O)OR", - NR"'C(O)NR'R", cycloalkyl, phenyl, naphthanelyl, 5,6,7,8-tetrahydronaphthanelyl, benzo[1,3]dioxolyl, heteroaryl or heterocyclyl; R', R" and R"' being as defined above; and wherein the cyclic groups present in group B are optionally substituted by one, two or three substituents selected from halogen, hydroxy, straight or branched, optionally substituted lower alkyl, phenyl, -OR', -SR', -NR'R", -NHCOR', -CONR'R", -CN, -NO₂ and - COOR'; R' and R" being as defined above;
X⁻ represents a pharmaceutically acceptable anion of a mono or polyvalent acid;
   including all individual stereoisomers of formulae (I) and mixtures thereof;

In the quaternary ammonium compounds of formula (I) an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, trifluoroacetate, maleate, fumarate, citrate, oxalate, succinate, tartrate, malate, mandelate, formate, methanesulfonate and p-toluenesulfonate. X⁻ is preferably an anion selected from chloride, bromide, iodide, sulphate, nitrate, acetate, trifluoroacetate, formate, methanesulfonate, maleate, oxalate or succinate. More preferably X⁻ is chloride, bromide, formate, trifluoroacetate or methanesulfonate.

The compounds of the present invention represented by formula (I), may have one or more asymmetric carbons. All possible stereoisomers are included, such as compounds of formula (I) wherein the carbon at the 3-position of the azoniabicyclic ring has either R or S configuration. All single isomers and mixtures of the isomers fall within the scope of the present invention.

Some of the carbamate derivatives that may be prepared following the process of the present invention have been described in the international patent applications WO02/00652, WO03/053966, WO02/051841 and WO2004/000840.

The above mentioned patent applications describe a process for preparing the compounds comprising the steps of obtaining a carbamate of an aminoalcohol of formula (II) followed by quaternisation of the carbamate to yield the quaternised compounds of formula (I).

Whilst the above-mentioned process is in some respects satisfactory, we have now devised an improved process allowing the manufacture of the compounds of formula (I) with greater yields.

According to the present invention, there is provided a process for preparing the compounds of formula (I) as hereinabove defined which process comprises the steps of:
a) Reacting a compound of formula (II) 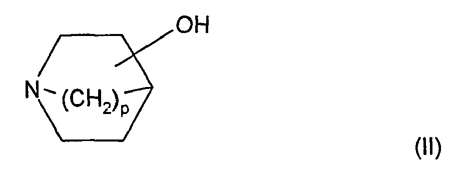 wherein p is as hereinabove defined with a compound of formula (III)

   B (CH₂)ₙ-A - (CH₂)ₘ-W (III)

   wherein A, B, n and m are as hereinabove defined, and wherein
   W is a leaving group such as a halogen or sulfonate ester, for example a Cl, Br or I atom or a methanesulfonate, or p-toluenesulfonate group,
   to yield a compound of formula (IV) 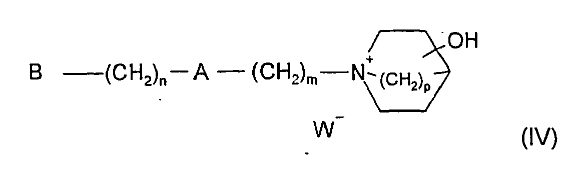
b) subsequently reacting the compound of formula (IV) with an acylating agent of formula (V), 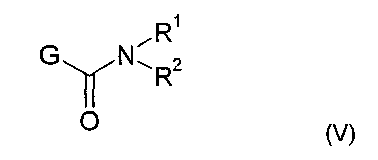 wherein G represents a leaving group such as:
   - an halogen atom such as chlorine or bromine,
   - a group of formula -OR^{IV} wherein R^{IV} is a lower alkyl group, or an optionally substituted phenyl group wherein the R^{IV} substitutent is preferably selected from the group consisting of methyl, ethyl, phenyl or 4-nitrophenyl group
   - an imidazol-1-yl group,
   - an O-succinimidyl group.
   and wherein R¹ and R² are as hereinabove defined
c) when W⁻ represents a group other than X⁻, exchanging the ion W in the product of step b) by the ion X⁻ using standard methods of anion exchange
to yield the quaternary ammonium salt of formula (I).

Thus, contrary to previous methods, the process of the invention proceeds first by quaternisation of the amino alcohols of formula (II) followed by acylation of the resulting quaternary ammonium alcohols (IV) to yield the compounds of formula (I). We have found that, surprisingly, by carrying out the process in the above manner the compounds are obtained in a greater yield.

In an embodiment of the present invention the leaving group G of the acylating agent of formula (IV) is selected from halogen atoms, preferably from chlorine or bromine atoms.

In another embodiment of the present invention the leaving group G of the acylating agent of formula (IV) is a group of formula -OR^{IV}, wherein R^{IV} is preferably selected from the group consisting of methyl, ethyl, phenyl or 4-nitrophenyl groups.

In still another embodiment of the present invention the reaction of step a) is performed by reflux in tetrahydrofurane.

In still another embodiment of the present invention the reaction of step b) is performed by addition of the compounds of formulae (IV) and (V) over a suspension of sodium hydride in dimethylformamide.

According to the present invention the first step of the synthetic pathway consists in the quaternisation of an amino alcohol of formula (II) with a compound of formula (III) to yield the compounds of formula (IV).

The amino alcohols of formula (II) are known compounds described in the prior art. See, for instance WO93/15080, Grob, C.A. et.al. Helv.Chim.Acta (1958), 41, 1184-1190, Ringdahl, R. Acta Pharm Suec. (1979), 16, 281-283 or commercially available from CU Chemie Uetikon GmbH.

The quaternising agents of formula (III) or their preparation processes have been described in the international patent application WO 2004/000840 A2.

An example of the synthetic methods which may be used to obtain compounds of formula (III) is described below.

Compounds of formula (III) wherein n = 0 and A= -O-, -S- or -NR', wherein R' is as defined above, may be obtained by reaction of the corresponding alcohol, thiol or amine derivative of formula B-AH or its sodium or potassium salt with an alkylating agent of general formula Y-(CH₂)m-W, wherein W and m are as herein above defined and Y is a leaving group such as a halogen atom or a sulphonate ester group. In other examples, compounds of general formula (III), where n>=1 were synthesised from the corresponding alcohol derivative of general formula (VII) by known methods.

B -(CH₂)ₙ- A - (CH₂)ₘ- OH (VII)

Quaternary ammonium derivatives of general formula (IV) may be prepared as is described in the following scheme (figure 1), by reaction of an alkylating agent of general formula (III) with the amino alcohols of formula (II). The synthesis is in particular performed as shown in figure 1.

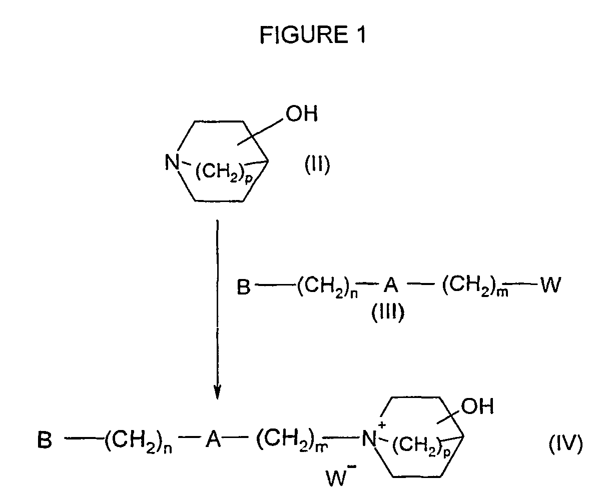

The reaction shown in figure 1 may be carried out in different solvents as for example an ether solvent such as THF, a chlorinated solvent such as CHCl₃, a ketone solvent such as acetone or methylisobutylketone (MIBK), DMSO, acetonitrile or mixed solvents thereof, at a temperature ranging from room temperature to reflux temperature of the solvent. In some cases and excess of alkylating agent may be used as a solvent.

The preferred conditions for the process shown in figure 1 are the following:

A mixture of compounds (II) and (III) was heated at reflux in tetrahydrofuran. After cooling at room temperature, the product of formula (IV) was isolated according to the methods described in the experimental section or other methods described in the art such as solid phase extraction methodologies or preparative HPLC/MS.

The present invention provides a second step in the manufacture of the compounds of formula (I) which consists in the reaction of the compounds of formula (IV) with derivatives of formula (V) as shown in figure 2.

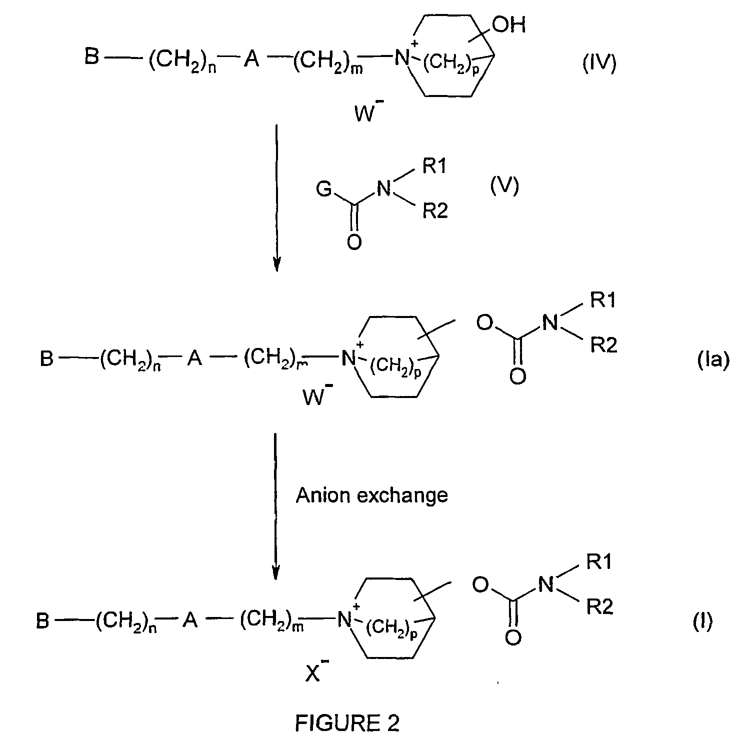

The process described in figure 2, wherein G represents a leaving group as defined previously, may be carried out in different conditions:

The reaction may be carried out in different organic solvents such as CHCl₃, CH₂Cl₂, 1,2-dichlororoethane, acetonitrile, toluene, dimethylformamide (DMF), tetrahydrofuran (THF), dioxane, dimethoxyethane, diethoxyethane, or mixed solvents thereof, at a temperature ranging from 0°C to the temperature of reflux of the solvent. The reaction can be carried out in the presence of a base, as for example sodium hydride, potassium hydride, sodium methoxide, sodium ethoxide, triethylamine, diisopropylethylamine, dimethylaminopyridine (DMAP) or DBU (1,8-diazabicyclo[5.4.0]undec-7-ene). As it is known in the art, when a base such as sodium hydride, potassium hydride, sodium methoxide or sodium ethoxide, is used, an inert solvent is required. Examples of inert solvents include dimethylformamide (DMF), tetrahydrofuran (THF), dioxane, dimethoxyethane, diethoxyethane, toluene or mixed solvents thereof.

Preferred conditions for the process shown in figure 2 are the following:

Over a suspension of a base such as sodium hydride in an inert solvent such as dimethylformamide, two separated solutions of compounds (IV) and (V), were simultaneously added slowly. The mixture was stirred at room temperature. Then water was added and the solvents were evaporated under reduced pressure. The residue was extracted with an organic solvent like for example dichloromethane and an aqueous solution containing a bromide salt such as potassium bromide (approximately 65% solution). The organic solvent was evaporated and the residue was purified following methods described in the art (for example by trituration with different organic solvents, purification by preparative HPLC/MS cromatography, or solid phase extraction methodologies).

If W⁻ represents a group other than X⁻, the quaternary ammonium salt of formula (I) is produced from the product of formula (Ia) obtained in the process shown in figure 2 by carrying out an anion exchange according to standard methods to replace the anion W⁻ with the desired anion X⁻, such as anion exchange reaction methods.

The compounds of general formula (V), wherein G is a leaving group as defined previously, may be prepared from the corresponding secondary amines of formula (VI) (figure 3) according to procedures described in literature.

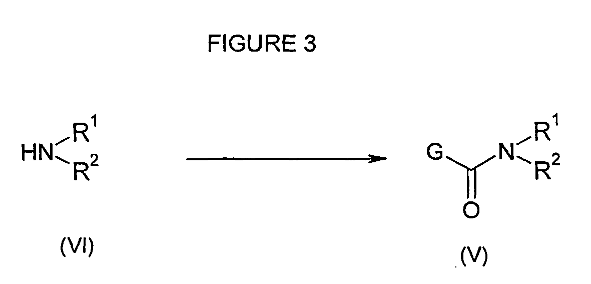

Compounds of formula (V) wherein G is a chlorine atom, may be prepared by reaction of the corresponding amine of formula (VI) with a phosgene source such as triphosgene as described in M. Saraswati et al. Drug Development Research (1994), 31, 142-146; G.M. Shutske et al., J. Heterocycl. Chem. (1990), 27, 1617; GB 1246606; US 2762796; WO 2002/051841 A1 or WO 2004/000840 A2.

Compounds of formula (V) wherein G is a chlorine or bromine atom, may be prepared according to the method described in N. Schindler et al, Chemische Berichte (1971), 104(3), 969-71.

Compounds of formula (V) wherein G is a group of formula -OR^{IV} for example a methoxy, ethoxy, phenoxy or substituted phenoxy group, may be prepared by methods known in the art, for example by reaction of the amine of formula (VI) with the corresponding chloroformate in an inert solvent such as CH₂Cl₂, 1,2-dichloroethane, THF, toluene or DMF in the presence of a base such as triethylamine, at a temperature ranging from 0°C to the temperature of reflux of the solvent as described, for example, in EP 0 801 067 B1.

Compounds wherein G is an imidazol-1-yl group may be prepared by methods known in the art, for example by reaction of the corrresponding amine of formula (VI) with N,N'-carbonyldiimidazole (CDI) in a solvent such as CH₂Cl₂, acetonitrile, DMF or THF. The reaction may be carried out in the presence of a base such as triethylamine or dimethylaminopyridine as described, for example, in L.A. Paquette, Encyclopedia of reagents for Organic Synthesis, Wiley, 1995, Volum 2, 1006-1010.

Compounds wherein G is an O-succinimidyl group may be prepared by reaction of the corresponding amine of formula (VI) with N,N'-disuccinimidyl carbonate (DSC) in a solvent such as acetonitrile or CH₂Cl₂ and in the presence of a base such as triethylamine or diisopropylethylamine as described, for example, in L.A. Paquette, Encyclopedia of reagents for Organic Synthesis, Wiley, 1995, Volum 4, 2304-5 or Takeda K. et al, Tetrahedron Letters, Vol 24, n° 42, pp 4569-4572, 1983.

Amines of general formula (VI) that are not commercially available may be prepared by synthesis according to standard methods, such as alkylation of anilines or reductive alkylation. For example, amines wherein R¹ is an optionally substituted thiophen-2-ylmethyl or an optionally substituted furan-2-ylmethyl and R² is as defined above, may be obtained by reductive alkylation. The corresponding aldehyde is treated with the corresponding primary amine to form the imine, which is reduced with sodium borohydride in methanol to obtain the secondary amine.

As used herein, an alkyl, alkenyl or alkynyl group or moiety can be straight or branched, and is typically a lower alkyl, alkenyl or alkynyl group. A lower alkyl group contains 1 to 8, preferably 1 to 6, carbon atoms. Examples include methyl, ethyl, propyl, including i-propyl, butyl, including n-butyl, sec-butyl and tert-butyl, 1-methylbutyl, 1-ethylpropyl, 1,2-dimethylpropyl, n-hexyl or 1-ethylbutyl groups. More preferably a lower alkyl group contains from 1 to 4 carbon atoms. A lower alkenyl or alkynyl group contains 2 to 8, preferably 2 to 6, carbon atoms. Examples include vinyl, allyl, 1-propenyl, 4-pentenyl, 1-propynyl, 2-propynyl, 1-butynyl, 2-butynyl or 3-butynyl groups. More preferably, a lower alkenyl or alkynyl group contains 2 to 4 carbon atoms.

Optionally substituted lower alkyl, alkenyl or alkynyl groups mentioned herein include straight or branched lower alkyl, alkenyl or alkynyl groups as defined above, which may be unsubstituted or substituted in any position by one or more substituents, for example by 1, 2 or 3 substituents. When two or more substituents are present, each substituent may be the same or different. The substituent(s) are typically halogen atoms, preferably fluoride atoms, and hydroxy or alkoxy groups.

Alkoxy and alkylthio groups mentioned herein are typically lower alkoxy and alkylthio groups, that is groups containing from 1 to 8, preferably 1 to 6 and more preferably 1 to 4 carbon atoms, the hydrocarbon chain being branched or straight and optionally substituted in any position by one or more substituents, for example by 1, 2 or 3 substituents. When two or more substituents are present, each substituent may be the same or different. The substituent(s) are typically halogen atoms, most preferably fluoride atoms, and hydroxy groups. Preferred optionally substituted alkoxy groups include methoxy, ethoxy, n-propoxy, i-propoxy, n-butoxy, sec-butoxy, t-butoxy, trifluoromethoxy, difluoromethoxy, hydroxymethoxy, 2-hydroxyethoxy or 2-hydroxypropoxy. Preferred optionally substituted alkylthio groups include methylthio, ethylthio, n-propylthio, i-propylthio, n-butylthio, sec-butylthio, t-butylthio, trifluoromethylthio, difluoromethylthio, hydroxymethylthio, 2-hydroxyethylthio or 2-hydroxypropylthio.

Cyclic groups mentioned herein include, unless otherwise specified, carbocyclic and heterocyclic groups. The cyclic groups can contain one or more rings. Carbocyclic groups may be aromatic or alicyclic, for example cycloalkyl groups. Heterocyclic groups also include heteroaryl groups.

Cycloalkyl groups and alicyclic groups mentioned herein, unless otherwise specified, typically contain from 3 to 7 carbon atoms. Cycloalkyl groups and alicyclic rings of 3 to 7 carbon atoms include cyclopropyl, cyclobutyl, cyclopentyl, cyclohexyl and cycloheptyl.

As used herein an aromatic group typically contains from 5 to 14, preferably 5 to 10 carbon atoms. Examples of aromatic groups include phenyl and naphthalenyl.

A heterocyclic or heteroaromatic group mentioned herein is typically a 5 to 10 membered group, such as a 5, 6 or 7 membered group, containing one or more heteroatoms selected from N, S and O. Typically, 1, 2, 3 or 4 heteroatoms are present, preferably 1 or 2 heteroatoms. A heterocyclic or heteroaromatic group may be a single ring or two or more fused rings wherein at least one ring contains a heteroatom. Examples of heterocyclic groups include piperidyl, pyrrolidyl, piperazinyl, morpholinyl, thiomorpholinyl, pyrrolyl, imidazolyl, imidazolidinyl, pyrazolinyl, indolinyl, isoindolinyl, pyridyl, pyrazinyl, pyrimidinyl, pyridazinyl, indolizinyl, isoindolyl, indolyl, indazolyl, purinyl, quinolizinyl, isoquinolyl, quinolyl, phthalazinyl, naphthyridinyl, quinoxalinyl, quinazolinyl, cinnolinyl, pteridinyl, quinuclidinyl, triazolyl, pyrazolyl, tetrazolyl and thienyl. Examples of heteroaromatic groups include pyridyl, thienyl, furyl, pyrrolyl, imidazolyl, benzothiazolyl, pyridinyl, pyrazolyl, pyrazinyl, pyrimidinyl, pyridazinyl, indolyl, indazolyl, purinyl, quinolyl, isoquinolyl, phthalazinyl, naphthyridinyl, quinoxalinyl, quinazolinyl, cinnolinyl, triazolyl and pyrazolyl.

As used herein a halogen atom includes a fluorine, chlorine, bromine or iodine atom, typically a fluorine, chlorine or bromine atom.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, formic, mandelic, ascorbic, oxalic, succinic, tartaric, benzoic, acetic, methanesulphonic, ethanesulphonic, benzenesulphonic or p-toluenesulphonic acid.

In the quaternary ammonium compounds that may be prepared following the process of the present invention an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, trifluoroacetate, maleate, fumarate, citrate, oxalate, succinate, tartrate, malate, mandelate, formate, methanesulfonate and p-toluenesulfonate. X⁻ 16 preferably an anion selected from chloride, bromide, iodide, sulphate, nitrate, acetate, trifluoroacetate, formate, methanesulfonate, maleate, oxalate or succinate. More preferably X⁻ is chloride, bromide, formate, trifluoroacetate or methanesulfonate.

Among the compounds that may be prepared following the process of the invention those where R¹ represents a group selected from phenyl, 2-furyl, 3-furyl, 2-thienyl, 3-thienyl, benzyl, furan-2-ylmethyl, furan-3-ylmethyl, thiophen-2-yl-methyl, thiophen-3-ylmethyl; the cyclic groups present in R¹ being optionally substituted by one, two or three substituents selected from halogen, straight or branched, optionally substituted lower alkyl, hydroxy, straight or branched, optionally substituted lower alkoxy, -SH, straight or branched optionally substituted lower alkylthio, nitro, cyano, -NR'R", -CO₂R', -C(O)-NR'R", - N(R"')C(O)-R', -N(R"')-C(O)NR'R", wherein R', R" and R"' each independently represents a hydrogen atom or a straight or branched, optionally substituted lower alkyl group or R' and R" together with the atom to which they are attached form a cyclic group, are preferred.

Also preferred is the preparation of compounds of formula (I) as defined above wherein R² represents an optionally substituted group selected from lower alkyl, lower alkenyl, lower alkynyl, saturated or unsaturated cycloalkyl, phenyl, benzyl, phenethyl, furan-2-ylmethyl, furan-3-ylmethyl, thiophen-2-ylmethyl, thiophen-3-ylmethyl, pyridyl, and pyridylmethyl or a saturated or unsaturated cycloalkylmethyl group which has at least one substituent and is selected from substituted cyclopropylmethyl, substituted cyclobutylmethyl and substituted cyclopentylmethyl; the substituents of the cyclic groups present in R² being one, two or three substituents selected from halogen, straight or branched, optionally substituted lower alkyl, hydroxy, straight or branched, optionally substituted lower alkoxy, -SH, straight or branched optionally substituted lower alkylthio, nitro, cyano, -NR'R", -CO₂R', - C(O)-NR'R", -N(R"')C(O)-R', -N(R"')-C(O)NR'R", wherein R', R" and R'" each independently represents a hydrogen atom or a straight or branched, optionally substituted lower alkyl group or R' and R" together with the atom to which they are attached form a cyclic group;

A further preferred embodiment is the preparation of compounds of formula (I) as defined above wherein R¹ represents a group selected from phenyl, 2-thienyl, 3-thienyl, thiophen-2-ylmethyl, thiophen-3-ylmethyl, furan-2-ylmethyl or furan-3-ylmethyl, the cyclic groups present in R¹ being optionally substituted with one to three substitutents selected from fluorine, chlorine, bromine, methyl, methoxy, trifluoromethyl, ethyl, tert-butyl, hydroxy and cyano.

Particularly preferred is the preparation of compounds of formula (I) wherein R¹ represents a group selected from phenyl, furan-2-ylmethyl, 2-fluorophenyl, 3-fluorophenyl, 4-fluorophenyl, 3-methylphenyl, 4-methylphenyl, 2,5-difluorophenyl, 2,6-difluorophenyl, 2,4,5-trifluorophenyl, 5-methylfuran-2-ylmethyl, 4-fluoro-2-methylphenyl, 3-fluoro-4-methoxyphenyl, 3-methyl-thiophen-2-ylmethyl, 4,5-dimethyl-thiophen-2-ylmethyl, thiophen-3-ylmethyl, 5-methyl-furan-2-ylmethyl, 5-methyl-2-trifluoromethyl-furan-3-ylmethyl, and 2,5-dimethyl-furan-3-ylmethyl,

Another preferred embodiment is the preparation of compounds of formula (I) as defined above wherein R² represents a pent-4-enyl, pentyl, butyl, allyl, benzyl, thiophen-2-ylmethyl, thiophen-3-ylmethyl, furan-2-ylmethyl, furan-3-ylmethyl, phenethyl, cyclopentyl, cyclohexyl or cyclohexylmethyl group, the cyclic groups present in R² being optionally substituted with one to three substitutents selected from fluorine, chlorine, bromine, methyl, methoxy, trifluoromethyl, ethyl, tert-butyl, hydroxy and cyano.

A particularly preferred embodiment is the preparation of compounds wherein R² represents a group selected from benzyl, tiophen-2-ylmethyl, 3-fluorobenzyl, 2,4,5-trifluorobenzyl, 3,4,5-trifluorobenzyl, 5-Bromothiophen-2-ylmethyl, 2-(3,4-dimethoxyphenyl)ethyl, 3-methylthiophen-2-ylmethyl, thiophen-3-ylmethyl, 4-bromo-5-methylthiophen-2-ylmethyl, 4,5-dimethylfuran-2-ylmethyl, furan-3-ylmethyl, 2-fluoro-4-methoxybenzyl, 2-(4-fluorophenyl)ethyl, butyl, pent-4-enyl and cyclopentyl.

A further preferred embodiment is the preparation of compounds of formula (I) wherein A is -CH₂-, m and n are both 0, and B represents a group selected from straight or branched, optionally substituted lower alkyl, hydroxy, straight or branched, optionally substituted lower alkoxy, cyano, nitro, -CH=CR'R", -C(O)OR', -OC(O)R', -SC(O)R', - C(O)NR'R", -NR'C(O)OR", -NR"'C(O)NR'R", cycloalkyl, phenyl, naphthalenyl, 5,6,7,8-tetrahydronaphthalenyl, benzo[1,3]dioxolyl, heteroaryl or heterocyclyl; R', R" and R"' being as defined above; and wherein the cyclic groups present in group B are optionally substituted by one, two or three substituents selected from halogen, hydroxy, straight or branched, optionally substituted lower alkyl, phenyl, -OR', -SR', -NR'R", -NHCOR', - CONR'R", -CN, -NO₂ and -COOR'; R' and R" being as defined above;

In other embodiments compounds of formula (I) wherein A is -CH₂-, B is as defined above and at least one of m or n is not 0, are prepared.

Also preferred is the preparation of compounds of formula (I) wherein B represents a thiophen-2-yl group or a phenyl group which is optionally substituted with one to three substituents selected from halogen atoms, or hydroxy, methyl, -CH₂OH -OMe, -NMe₂, - NHCOMe, -CONH₂, -CN, -NO₂, -COOMe, or -CF₃ groups. Most preferred are compounds wherein B represents a phenyl, 4-fluorophenyl, 3-hydroxyphenyl or thiophen-2-yl group.

In particularly preferred embodiments the compounds of formula (I) wherein n= 0 or 1; m is an integer from 1 to 6; and A represents a -CH₂-, -CH=CH-, -CO-, -NMe-, -O- or -S-group, are prepared. Most preferred is the preparation of compounds wherein m is 1, 2 or 3 and A represents a -CH₂-, -CH=CH-, or -O- group.

Preferably, the compounds of formula (I) wherein the sequence B-(CH₂)ₙ-A-(CH₂)ₘ- represents a group selected from 3-phenoxypropyl, 2-phenoxyethyl, 3-phenylallyl, phenethyl, 3-phenylpropyl, 3-(3-hydroxyphenoxy)propyl, 3-(4-fluorophenoxy)propyl, 3-thiophen-2-ylpropyl, allyl, heptyl, 3-cyanopropyl, 2-ethoxyethyl, 2-(2-methoxyethoxy)ethyl and methyl, are prepared.

It is preferred to use the process of the invention for the preparation of compounds of formula (I) wherein X- represents a chloride, bromide, formate, trifluoroacetate or methanesulphonate anion.

It is also preferred to use the process of the invention for the preparation of compounds of formula (I) wherein p is 2 and/or wherein the azoniabicyclic ring is substituted in the 3-position.

The process of the present invention may be used to prepared compounds of formula (I) having one or more asymmetric carbons. All possible stereoisomers may be prepared, such as compounds of formula (I) wherein the carbon at the 3-position of the azoniabicyclic ring has either R or S configuration. The preparation of all single isomers and mixtures of the isomers fall within the scope of the present invention.

The process of the present invention may be used in particular for the preparation of the following compounds:
3-(R)(Benzylphenylcarbamoyloxy)-1-(3-phenylallyl)-1-azoniabicyclo[2.2.2]octane; bromide
1-Allyl-3-(R)(benzylphenylcarbamoyloxy)-1-azoniabicyclo[2.2.2]octane; bromide
3-(R)(Benzylphenylcarbamoyloxy)-1-phenethyl-1-azoniabicyclo[2.2.2]octane;
bromide
3-(R)(Benzylphenylcarbamoyloxy)-1-(3-thiophen-2-yl-propyl)-1-azoniabicyclo[2.2.2] octane; bromide
3-(R)(Benzylphenylcarbamoyloxy)-1-(2-phenoxyethyl)-1-azoniabicyclo[2.2.2]octane; bromide
1-Allyl-3-(R)(butylphenylcarbamoyloxy)-1-azoniabicyclo[2.2.2]octane; bromide
3-(R)(Butylphenylcarbamoyloxy)-1-(2-phenoxyethyl)-1-azoniabicyclo[2.2.2]octane; bromide
3-(R)(Butylphenylcarbamoyloxy)-1-[3-(3-hydroxyphenoxy)propyl]-1-azoniabicyclo [2.2.2]octane; bromide
3-(R)(Butylphenylcarbamoyloxy)-1-[3-(4-fluorophenoxy)propyl]-1-azoniabicyclo[2.2.2] octane; bromide
3-(R)(Butylphenylcarbamoyloxy)-1-(3-phenylpropyl)-1-azoniabicyclo[2.2.2]octane; bromide
3-(R)(Phenylthiophen-2-ylmethylcarbamoyloxy)-1-(3-thiophen-2-ylpropyl)-1-azonia bicyclo[2.2.2]octane; bromide
1-(2-Phenoxy-ethyl)-3-(R)-(phenyl-thiophen-2-ylmethyl-carbamoyloxy)-1-azoniabicyclo [2.2.2]octane; bromide
1-Allyl-3-(R)(phenylthiophen-2-ylmethylcarbamoyloxy)-1-azoniabicyclo[2.2.2]octane; bromide
3-(R)(Phenethylphenylcarbamoyloxy)-1-(2-phenoxyethyl)-1-azoniabicyclo[2.2.2]octane; trifluoroacetate
1-Heptyl-3-(R)(pent-4-enylphenylcarbamoyloxy)-1-azoniabicyclo[2.2.2]octane; trifluoroacetate
1-Allyl-3-(R)-(phenyl-thiophen-3-ylmethl-carbamoyloxy)-1-azoniabicyclo[2.2.2]octane; trifluoroacetate
3-(R)(phenylthiophen-3-ylmethylcarbamoyloxy)-1-(3-thiophen-2-ylpropyl)-1-azonia bicyclo[2.2.2]octane; bromide
1-(2-Phenoxyethyl)-3-(R)(phenylthiophen-3-ylmethylcarbamoyloxy)-1-azoniabicyclo [2.2.2]octane; bromide
3-(R)(Bis-thiophen-2-ylmethylcarbamoyloxy)-1-(3-phenylpropyl)-1-azoniabicyclo [2.2.2]octane; bromide
3-(R)(Bis-thiophen-2-ylmethylcarbamoyloxy)-1-(3-thiophen-2-ylpropyl)-1-azoniabicyclo [2.2.2]octane; bromide
1-Allyl-3-(R)(allylthiophen-2-ylmethylcarbamoyloxy)-1-azoniabicyclo[2.2.2]octane; trifluoroacetate
3-(R)(Cyclopentylthiophen-2-ylmethylcarbamoyloxy)-1-(3-phenylpropyl)-1-azonia bicyclo[2.2.2]octane; trifluoroacetate
3-(R)(Furan-2-ylmethylphenylcarbamoyloxy)-1-(3-phenylpropyl)-1-azoniabicyclo [2.2.2]octane; trifluoroacetate
1-Allyl-3-(R)(bis-furan-2-ylmethylcarbamoyloxy)-1-azoniabicyclo[2.2.2]octane; trifluoroacetate
(3R)-3-(Benzylphenylcarbamoyloxy)-1-(3-phenylpropyl)-1-azoniabicyclo[2.2.2]octane; bromide
(3R)-3-(Butylphenylcarbamoyloxy)-1-(3-phenylallyl)-1-azoniabicyclo[2.2.2]octane; bromide
(3R)-3-(Butylphenylcarbamoyloxy)-1-(3-thiophen-2-ylpropyl)-1-azoniabicyclo[2.2.2] octane; bromide
(3R)-3-[(3-Fluorobenzyl)-(3-fluorophenyl)carbamoyloxy]-1-(2-phenoxyethyl)-1-azoniabicyclo[2.2.2]octane bromide
(3R)-3-[(3-Fluorobenzyl)-(3-fluorophenyl)carbamoyloxy]-1-(3-phenylpropyl)-1-azoniabicyclo[2.2.2]octane bromide
(3R)-1-(2-Phenoxyethyl)-3-[m-tolyl-(2,4,5-trifluorobenzyl)carbamoyloxy]-1-azoniabicyclo[2.2.2]octane bromide
(3R)-1-(3-Phenylpropyl)-3-[m-tolyl-(2,4,5-trifluorobenzyl)carbamoyloxy]-1-azoniabicyclo[2.2.2]octane bromide
(3R)-3-[(3-Fluorophenyl)-(3,4,5-trifluorobenzyl)carbamoyloxy]-1-(2-phenoxyethyl)-1-azoniabicyclo[2.2.2]octane bromide
(3R)-1-Allyl-3-[[2-(3,4-dimethoxyphenyl)ethyl]-(5-methylfuran-2-ylmethyl)carbamoyloxy]-1-azoniabicyclo[2.2.2]octane bromide
(3R)-3-[(5-Bromothiophen-2-ylmethyl)-(2,4,5-trifluorophenyl)carbamoyloxy]-1-(3-phenoxypropyl)-1-azoniabicyclo[2.2.2]octane trifluoroacetate
(3R)-3-[[2-(3,4-dimethoxyphenyl)ethyl]-(5-methylfuran-2-ylmethyl)carbamoyloxy]-1-(4-ethoxycarbonylbutyl)-1-azoniabicyclo[2.2.2]octane trifluoroacetate
(3R)-3-[(4-Fluoro-2-methylphenyl)-(3-methylthiophen-2-ylmethyl)carbamoyloxy]-1-(2-phenoxyethyl)-1-azoniabicyclo[2.2.2]octane trifluoroacetate
(3R)-3-[(3-Fluoro-4-methoxyphenyl)thiophen-3-ylmethylcarbamoyloxy]-1-(3-phenylallyl)-1-azoniabicyclo[2.2.2]octane trifluoroacetate
(3R)-1-Phenethyl-3-[thiophen-3-ylmethyl-(2,4,5-trifluorobenzyl)carbamoyloxy]-1-azoniabicyclo[2.2.2]octane trifluoroacetate
(3R)-3-[(4-Bromo-5-methylthiophen-2-ylmethyl)-(3-methylthiophen-2-ylmethyl)carbamoyloxy]-1-(3-phenylpropyl)-1-azoniabicyclo[2.2.2]octane trifluoroacetate
(3R)-3-[(4,5-Dimethylfuran-2-ylmethyl)-(5-methylfuran-2-ylmethyl)carbamoyloxy]-1-[3-(3-hydroxyphenoxy)propyl]-1-azoniabicyclo[2.2.2]octane trifluoroacetate
(3R)-1-[3-(4-Fluorophenoxy)propyl]-3-[furan-3-ylmethyl-(5-methyl-2-trifluoromethylfuran-3-ylmethyl)carbamoyloxy]-1-azoniabicyclo[2.2.2]octane trifluoroacetate
(3R)-3-[(2,5-Dimethylfuran-3-ylmethyl)-(2-fluoro-4-methoxybenzyl)carbamoyloxy]-1-(3-thiophen-2-ylpropyl)-1-azoniabicyclo[2.2.2]octane trifluoroacetate
(3R)-1-Allyl-3-[2-(4-fluorophenyl)ethyl]-(3-methylthiophen-2-ylmethyl)carbamoyloxy]-1-azoniabicyclo[2.2.2]octane trifluoroacetate
(3R)-3-[Butyl-(2,5-difluorophenyl)carbamoyloxy]-1-heptyl-1-azoniabicyclo[2.2.2]octane trifluoroacetate
(3R)-1-(3-cyanopropyl)-3-[(2,6-difluorophenyl)pent-4-enylcarbamoyloxy]-1-azoniabicyclo[2.2.2]octane trifluoroacetate
(3R)-3-[Cyclopentyl-(4,5-dimethylthiophen-2-ylmethyl)carbamoyloxy]-1-methyl-1-azoniabicyclo[2.2.2]octane trifluoroacetate
(3R)-3-[(3-Fluorophenyl)-(3,4,5-trifluorobenzyl)carbamoyloxy]-1-(3-phenylpropyl)-1-azoniabicyclo[2.2.2]octane bromide
(3R)-3-[(5-Ethylthiophen-2-ylmethyl)-(3-methylthiophen-2-ylmethyl)carbamoyloxy]-1-(3-phenylpropyl)-1-azoniabicyclo[2.2.2]octane bromide
(3R)-3-[[2-(3,4-dimethoxyphenyl)ethyl]-(5-methylfuran-2-ylmethyl)carbamoyloxy]-1-(4-ethoxycarbonylbutyl)-1-azoniabicyclo[2.2.2]octane formate
(3R)-3-[(4-Fluoro-2-methylphenyl)-(3-methylthiophen-2-ylmethyl)carbam oyloxy]-1-(2-phenoxyethyl)-1-azoniabicyclo[2.2.2]octane bromide
(3R)-3-[(3-Fluoro-4-methoxyphenyl)thiophen-3-ylmethylcarbamoyloxy]-1-(3-phenylallyl)-1-azoniabicyclo[2.2.2]octane bromide
(3R)-1-Allyl-3-[2-(4-fluorophenyl)ethyl]-(3-methylthiophen-2-ylmethyl)carbamoyloxy]-1-azoniabicyclo[2.2.2]octane bromide
(3*R*)-3-[[2-(3,4-Dimethoxyphenyl)ethyl]-(5-methylfuran-2-ylmethyl)carbamoyloxy]-1-heptyl-1-azoniabicyclo [2.2.2]octane bromide
(3*R*)-1-(2-Ethoxyethyl)-3-(furan-2-ylmethylthiophen-2-ylmethylcarbamoyloxy)-1-azoniabicyclo [2.2.2]octane formate
(3*R*)-3-(Furan-2-ylmethylthiophen-2-ylmethylcarbamoyloxy)-1-[2-(2-methoxyethoxy)ethyl]-1-azoniabicyclo [2.2.2]octane formate

The methods of synthesis described in the present invention will be further illustrated by the following examples. The examples are given by the way of illustration only and are not to be construed as limiting.

The structures of the prepared compounds were confirmed by ¹H-NMR and MS. NMR were recorded using a Varian Gemini-200 NMR spectrometer operating at frequency of 200 or 300 MHz. Tetramethyl silane was used as a reference and samples were solved in deuterated methylsulphoxide (DMSO-d₆) or deuterated chloroform (CDCl₃).
Their purity was determined by HPLC, in Alliance 2795 Waters instrument equipped with diode array detector (DAD) and ZMD or ZQ mass detector (electrospray ionization). HPLC method used a Symmetry C18 column (3.5 µm, 21x100 mm) and mobile phase was composed by two phases: Phase A: Buffered (Formic acid/ammonia) aqueous solution at pH: 3. Phase B: 50.50 mixture acetonitrile/methanol with ammonia formiate. Gradient was from 0% to 95% of phase B in 10 minutes.

Preparative HPLC-MS experiments were performed on a Gilson instrument equipped with a binary pump (Gilson piston pump 321); a vacuum degasser (Gilson 864); an injector-fraction collector (Gilson liquid handler 215); two injection modules, analytical and preparative (Gilson 819); a valve (Gilson Valvemate 7000); a 1/1000 splitter (Acurate by LC Packings); a make-up pump (Gilson 307); a diode array detector (Gilson 170) and a MS detector (a Thermoquest Finnigan aQa, a quadrupole mass spectrometer with ES an APCI ionisation modes). The HPLC-MS instrument was controlled by an IBM PC.

The following intermediate carbamoyl chlorides of general formula (V) have been prepared following the method depicted in figure 3 following procedures described in the literature: M. Saraswati et al. Drug Development Research (1994), 31, 142-146; G.M. Shutske et al., J. Heterocycl. Chem. (1990), 27, 1617; GB 1246606; US 2762796; WO 2002/051841 A1; WO 2004/000840 A2.

### Intermediate I-1

### Benzylphenylcarbamoyl chloride

To a solution of 50 g (0.27 mol) of benzylphenylamine in 350 mi of methylisobutylketone was added slowly with stirring 40 g (0.13 mol) of triphosgene in 260 ml of methylisobutylketone keeping the temperature below 25°C. The reaction was allowed to continue at room temperature for 20 hours. The solvent was concentrated in vacuo to yield 75.5 g (100%) of the title compound.
[M+1]⁺: 246

Benzylphenylamine is commercially available.

### Intermediate I-2

### Butylphenylcarbamoyl chloride

To a solution of 5 g (33 mmol) of butylaniline in 35 ml of methylene chloride was added slowly with stirring 5 g (17 mmol) of triphosgene in 35 ml of methylene chloride keeping the temperature below 25°C. The reaction was refluxed for 6 hours. The solvent was concentrated in vacuo to yield 7.7 g of a residue. This residue was purified by filtration through silica gel using ethyl acetate as eluant to obtain 7.0 g of a pure product (99%).
¹H-NMR (DMSO-d₆): δ 0.9 (m, 3H), 1.3 (m, 2H), 1.6 (m, 2H), 3.7 (m, 2H), 7.2-7.4 (m, 5H).
MS [M+1]⁺: 212

Butylaniline is commercially available.

### Intermediate I-3

### Furan-2-ylmethylthiophen-2-ylmethylcarbamoyl chloride

The title compound was synthesised according to the process described for Intermediate I-2. The yield was 2.81 g, 70%.
¹H-NMR (DMSO-d₆): δ 4.56-4.58 (m, 4H), 6.45-6.46 (m, 2H), 6.99-7.10 (m, 2H), 7.50 (m, 1H), 7.66 (m, 1H).
MS [M+₁]⁺: 256
Furan-2-ylmethylthiophen-2-ylmethylamine was prepared by reductive alkylation from thiophene-2-carbaldehyde and 2-furfurylamine following standard conditions. This amine is also commercially available.

### Intermediate I-4

### 2-[(3,4-Dimethoxyphenyl)ethyl]-(5-methylfuran-2-ylmethyl)carbamoyl chloride

The title compound was synthesised according to the process described for Intermediate I-2. The yield of the step was 2.1 g, 49%.
¹H-NMR (DMSO-d₆): δ 2.26 (s, 3H), 2.69-2.77 (m, 2H), 3.50-3.59 (m, 2H), 3.72 (s, 3H), 3.74 (s, 3H), 4.50 (s, 1 H), 4.60 (s, 1H), 6.07(m, 1 H), 6.35 (m, 1 H), 6.69-6.89 (m, 3H). MS
[M+1]⁺: 338

2-[(3,4-Dimethoxyphenyl)ethyl]-(5-methylfuran-2-ylmethyl)amine⁽¹⁾ was prepared by reductive alkylation from 2-(3,4-dimethoxyphenyl)ethylamine and 5-methylfuran-2-carbaldehyde following standard conditions.

⁽¹⁾2-[(3,4-Dimethoxyphenyl)ethyl]-(5-methylfuran-2-ylmethyl)amine has been described in WO 2004/000840 A2 as I-5.

### Intermediate I-5

### m-Tolyl-(2,4,5-trifluorobenzyl)carbamoyl chloride

The title compound was synthesised according to the process described for Intermediate 1-2. The yield of the step was 6.2 g, 90%.
MS [M+1]⁺: 314

*m*-Tolyl-(2,4,5-trifluorobenzyl)amine⁽²⁾ was prepared by reductive alkylation from m-tolylamine and 2,4,5-trifluorobenzaldehyde following standard conditions.

⁽²⁾*m*-Tolyl-(2,4,5-trifluorobenzyl)amine has been described in WO 2004/000840 A2 as I-7.

The following intermediate compounds of general formula (IV) have been prepared following the method depicted in figure 1.

### Intermediate I-6

### (3R)-3-Hydroxy-1-(3-phenylpropyl)-1-azoniabicyclo[2.2.2]octane bromide

15 g (118 mmol) of (3R)-3-hydroxy-1-azabicyclo[2.2.2]octane, 34.5 g of 1-bromo-3-phenylpropane (173 mmol) and 225 ml of tetrahydrofuran were mixed. The mixture was refluxed for approximately 7 hours. After cooling to room temperature the mixture was filtered off and dried in a vacuum oven to give 38.6 g of a pure product (100%).
¹H-NMR (DMSO-d₆): δ 1.70-2.11 (m, 6H), 2.51-2.61 (m, 2H), 3.04 (m, 1H), 3.19-3.40 (m, 7H), 3.68 (m, 1H), 4.01 (m, 1H), 5.58 (d, 1H, J= 3.6 Hz), 7.19-7.34 (m, 5H).
MS [M-Br]⁺: 246.

### Intermediate I-7

### (3R)-3-Hydroxy-1-(3-phenylallyl)-1-azoniabicyclo[2.2.2]octane bromide

The title compound was synthesised according to the method described for Intermediate I-6. The yield was 5.4 g, 96%.
¹H-NMR (DMSO-d₆): δ 1.74-2.14 (m, 4H), 3.08 (m, 1H), 3.29-3.44 (m, 5H), 3.70 (m, 1 H), 4.01-4.10 (m, 3H), 5.58 (d, 1 H, J= 3.3 Hz), 6.49 (m, 1 H), 6.86 (d, 1H, J= 15.6 Hz), 7.35-7.44 (m, 3H), 7.57-7.60 (m, 2H).
MS [M-Br]⁺: 244.

### Intermediate 1-8

### (3R)-3-Hydroxy-1-(3-thiophen-2-ylpropyl)-1-azoniabicyclo[2.2.2]octane bromide

The title compound was synthesised according to the method described for Intermediate I-6. The yield was 2.8 g, 97%.
¹H-NMR (DMSO-d₆): δ 1.60-2.15 (m, 6H), 2.70-2.85 (m, 2H), 3.10 (m, 1 H), 3.18-3.40 (m, 7H), 3.65 (m, 1 H), 4.10 (m, 1H), 5.59 (d, 1H, J= 3.5 Hz), 6.85-7.10 (m, 2H), 7.35 (m, 1H).
MS [M-Br]⁺: 252.

### Intermediate I-9

### (3R)-1-Heptyl-3-hydroxy-1-azoniabicyclo[2.2.2]octane bromide

The title compound was synthesised according to the method described for Intermediate I-6. The yield was 9.1 g, 86%.
¹H-NMR (DMSO-d₆): δ 0.85-0.89 (m, 2H), 1.10-1.27 (m, 8H), 1.60-2.11 (m, 7H), 2.99 (m, 1 H), 3.12-3.35 (m, 7H), 3.64 (m, 1 H), 4.05 (m, 1 H), 5.56 (d, 1H, J= 3.3 Hz).
MS [M-Br]⁺: 226.

### Intermediate I-10

### (3R)-1-(2-Ethoxyethyl)-3-hydroxy-1-azoniabicyclo[2.2.2]octane bromide

The title compound was synthesised according to the method described for Intermediate I-6. The yield was 7.1 g, 100%.
¹H-NMR (DMSO-d₆): δ 1.13 (t, 3H, J= 8 Hz, J'= 14 Hz), 1.72-2.11 (m, 4H), 3.14 (m, 1H), 3.31-3.53 (m, 9H), 3.70-3.79 (m, 3H), 4.05 (m, 1 H), 5.58 (d, 1H, J= 4 Hz).
MS [M-Br]⁺: 200.

### Intermediate I-11

### (3R)-3-Hydroxy-1-[2-(2-methoxyethoxy)ethyl]-1-azoniabicyclo[2.2.2]octane bromide

1.4 g (11 mmol) of (3R)-3-hydroxy-1-azabicyclo[2.2.2]octane, 2.5 g of 1-bromo-2-(2-methoxyethoxy)ethane (13.7 mmol) and 35 ml of tetrahydrofuran were mixed. The mixture was refluxed for approximately 16 hours. After cooling to room temperature the solvent was decanted and isopropyl ether was added. The mixture was stirred for approximately 10 minutes and then the solvent was decanted. The same operation was repeated and finally methylene chloride was added. The solvent was evaporated and the residue was dried in a vacuum oven to give 3.7 g of a pure product (100%).
¹H-NMR (DMSO-d₆): δ 1.72-2.12 (m, 4H), 3.14 (m, 1H), 3.26-3.58 (m, 14H), 3.69-3.82 (m, 3H), 4.07 (m, 1 H), 5.57 (d, 1H, J= 3.6 Hz).
MS [M-Br]⁺: 230.

The following compounds of general formula (I) have been prepared following the method depicted in figure 2.

### Example 1

### (3R)-3-(Benzylphenylcarbamoyloxy)-1-(3-phenylpropyl)-1-azoniabicyclo[2.2.2] octane bromide

0.34 g (8.5 mmol) of sodium hydride, 60% dispersion in mineral oil, and 10 ml of dimethylformamide were mixed. Simultaneously two solutions of 2.8 g of (3R)-3-hydroxy-1-(3-phenylpropyl)-1-azoniabicyclo[2.2.2]octane bromide (Intermediate I-6) (8.6 mmol) in 40 ml of dimethylformamide and 2.0 g of benzylphenylcarbamoyl chloride (Intermediate I-1) (8.1 mmol) in 40 ml of dimethylformamide were added slowly. The mixture was stirred at room temperature for 3 hours. Water was added and the solvents were evaporated under reduced pressure. The residue was extracted with dichloromethane and water containing potassium bromide (65% solution). This operation was repeated twice. The organic solvent was evaporated and the residue was treated with 5 ml of tetrahydrofuran and 100 ml of methylisobutylketone. The resulting suspension was stirred at room temperature for 2.5 hours. The solid was filtered off, washed with 10 ml of methylisobutylketone and dried in a vacuum oven to give 2.0 g of a pure product (46%). ¹H-NMR (DMSO-d₆): δ 1.40-2.00 (m, 6H), 2.18 (bs, 1 H), 2.59 (m, 2H), 2.95-3.44 (m, 7H), 3.84 (m, 1H), 4.92 (s, 2H), 5.00 (m, 1H), 7.19-7.36 (m, 15H).
MS [M-Br]⁺: 455; mp: 101°C; Bromide content: 99.3%.

### Example 2

### (3R)-3-(Butylphenylcarbamoyloxy)-1-(3-phenylallyl)-1-azoniabicyclo[2.2.2]octane bromide

The title compound was synthesised according to the procedure described in Example 1 starting from butylphenylcarbamoyl chloride (Intermediate I-2) and (3R)-3-hydroxy-1-(3-phenylallyl)-1-azoniabicyclo[2.2.2]octane bromide (Intermediate I-7). The obtained oil product was treated with n-hexane at room temperature overnight. The resulting solid was filtered off and dried in a vacuum oven to give 1.7 g of a pure product (42%).
¹H-NMR (DMSO-d₆): δ 0.84 (m, 3H), 1.25 (m, 2H), 1.40 (m, 2H), 1.70-1.91 (m, 4H), 2.20 (s, 1 H), 3.2-3.4 (m, 6H), 3.64 (m, 2H), 3.88-4.07 (d, 2H), 4.97 (m, 1 H), 6.45 (m, 1 H), 6.83-6.88 (d, 1H), 7.23-7.45 (m, 7H), 7.60 (m, 2H).
MS [M-Br]⁺: 419; mp: 144°C; Bromide content: 100.5%.

### Example 3

### (3R)-3-(Butylphenylcarbamoyloxy)-1-(3-thiophen-2-ylpropyl)-1-azoniabicyclo[2.2.2] octane bromide

0.50 g (12.5 mmol) of sodium hydride, 60% dispersion in mineral oil, and 12 ml of dimethylformamide were mixed. Simultaneously two solutions of 2.5 g of (3R)-3-hydroxy-1-(3-thiophen-2-ylpropyl)-1-azoniabicyclo[2.2.2]octane bromide (Intermediate I-8) (7.5 mmol) in 35 ml of dimethylformamide and 1.5 g of butylphenylcarbamoyl chloride (Intermediate I-2) (7.1 mmol) in 35 ml of dimethylformamide were added slowly. The mixture was stirred at room temperature for 2 hours. Water was added and the solvents were evaporated under reduced pressure. The residue was extracted with dichloromethane from water containing potassium bromide (65% solution). This operation was repeated twice. The organic solvent was evaporated and the residue was treated with 16 ml of methylisobutylketone, 130 ml of hexane and 15 ml of cyclohexane. The resulting suspension was stirred at room temperature for 30 minutes. The solid was filtered off and dried in a vacuum oven to give 2.48 g of a pure product (70%).
¹H-NMR (DMSO-d₆): δ 0.84 (m, 3H), 1.24-1.31 (m, 2H), 1.42 (m, 2H), 1.60-2.21 (m, 7H), 2.85 (m, 2H), 3.0-3.50 (m, 7H), 3.60-3.69 (m, 2H), 3.85 (m, 1 H), 4.93 (m, 1 H), 6.95-7.00 (m, 2H), 7.28-7.43 (m, 6H).
MS [M-Br]⁺: 427; mp: 127°C; Bromide content: 99.8%.

### Example 4

### (3R)-1-(3-Phenylpropyl)-3-[m-tolyl-(2,4,5-trifluorobenzyl)carbamoyloxy]-1-azonia-bicyclo[2.2.2]octane bromide

The title compound was synthesised according to the procedure described in Example 1 starting from (3R)-3-hydroxy-1-(3-phenylpropyl)-1-azoniabicyclo[2.2.2]octane bromide (Intermediate I-6) and m-tolyl-(2,4,5-trifluorobenzyl)carbamoyl chloride (Intermediate I-5). The obtained oil product was treated with diisopropyl ether at room temperature overnight. The resulting solid was filtered off and dried in a vacuum oven to give 0.6 g of a pure product (32%).
¹H-NMR (DMSO-d₆): δ 1.40-1.60 (m, 1H), 1.60-1.80 (m, 1H), 1.80-2.10 (m, 4H), 2.18 (m, 1 H), 2.26 (s, 3H), 2.60 (t, 2H), 3.05-3.55 (m, 7H), 3.80-3.90 (m, 1 H), 4.90 (m, 2H), 4.98 (m, 1 H), 7.00-7.15 (m, 2H), 7.15-7.40 (m, 7H), 7.40-7.60 (m, 2H).
MS [M-Br]⁺: 523; mp: 113.1-114.8°C; Bromide content: 96.3%.

### Example 5

### (3R)-3-[[2-(3,4-Dimethoxyphenyl)ethyl]-(5-methylfuran-2-ylmethyl)carbamoyloxy]-1-heptyl-1-azoniabicyclo [2.2.2]octane bromide

The title compound was synthesised according to the procedure described in Example 1 starting from 2-[(3,4-Dimethoxyphenyl)ethyl]-(5-methylfuran-2-ylmethyl)carbamoyl chloride (Intermediate I-4) and (3R)-1-heptyl-3-hydroxy-1-azoniabicyclo[2.2.2]octane bromide (Intermediate I-9). The resulting oil is dissolved in methylisobutyl ketone and treated with n-hexane at room temperature for one hour. The resulting solid was filtered off and dried in a vacuum oven to give 1.8 g of a pure product (59%).
¹H-NMR (DMSO-d₆): δ 0.86 (m, 3H), 1.28 (m, 8H), 1.62 (m, 2H), 1.85-2.06 (m, 4H), 2.24-2.28 (m, 4H), 2.66-2.72 (m, 2H), 3.09-3.50 (m, 9H), 3.65-3.83 (m, 7H), 4.36 (m, 2H), 4.91 (m, 1 H), 6.03 (s, 1 H), 6.27 (s, 1H), 6.65-6.88 (m, 3H).
MS [M-Br]⁺: 527; mp: 121.0-121.9°C; Bromide content: 95.3%.

### Example 6

### (3R)-1-(2-Ethoxyethyl)-3-(furan-2-ylmethylthiophen-2-ylmethylcarbamoyloxy)-1-azoniabicyclo [2.2.2]octane formate

The title compound was synthesised according to the procedure described in Example 1, starting from furan-2-ylmethyl-thiophen-2-ylmethylcarbamoyl chloride (Intermediate I-3) and (3R)-1-(2-ethoxyethyl)-3-hydroxy-1-azoniabicyclo[2.2.2]octane bromide (intermediate I-10), the reaction product was purified by preparative HPLC/MS to give 53 mg of the formate.
¹H-NMR (CDCl₃): δ 1.17 (t, 3H), 2.0 (m, 2H), 2.20 (m, 2H), 2.52 (m, 1H), 3.20-4.15 (m, 12H), 4.39 (m, 1 H), 4.51 (m, 1 H), 4.63 (m, 2H), 5.12 (m, 1H), 6.18-6.32 (m, 1 H), 6.35 (m, 1 H), 6.89-6.98 (m, 2H), 7.27 (m, 1H), 7.39 (m, 1H), 8.59 (s, 1H).
MS [M-HCOO]⁺: 419.
Conditions used in the purification HPLC/MS:
Column: Symmetry C18, 100 A, 5 µm 19 x 100 mm, Waters.
Mobile phase: A (H₂O 0.1% HCOONH₄, pH=3) and B (AcN 0.1% HCOONH₄, pH=3), from 20% B to 45% B in 12 min.

### Example 7

### (3R)-3-(Furan-2-ylmethylthiophen-2-ylmethylcarbamoyloxy)-1-[2-(2-methoxyethoxy)ethyl]-1-azoniabicyclo [2.2.2]octane formate

The title compound was synthesised according to the procedure described in Example 1, starting from furan-2-ylmethyl-thiophen-2-ylmethylcarbamoyl chloride (Intermediate I-3) and (3R)-3-hydroxy-1-[2-(2-methoxyethoxy)ethyl]-1-azoniabicyclo[2.2.2]octane bromide (Intermediate I-11). The reaction product was purified by preparative HPLC/MS to give 37 mg of the formate.
¹H-NMR (CDCl₃): δ 2.01 (m, 2H), 2.17 (m, 2H), 2.50 (m, 1H), 3.33 (s, 3H), 3.40-4.10 (m, 14H), 4.39 (m, 1H), 4.42-4.65 (m, 3H), 5.13 (m, 1H), 6.19-6.31 (m, 1H), 6.35 (m, 1H), 6.90-6.98 (m, 2H), 7.27 (m, 1 H), 7.40 (m, 1H), 8.54 (s, 1H).
MS [M-HCOO]⁺: 449.
Conditions used in the purification HPLC/MS:
Column: Symmetry C18, 100 A, 5 µm 19 x 100 mm, Waters.
Mobile phase: A (H₂O 0.1 % HCOONH₄, pH=3) and B (AcN 0.1 % HCOONH₄, pH=3), from 10% B to 25% B in 12 min.

The compounds of examples 1 to 4 have been prepared according to the method of the present invention and also according to the method described in international patent application WO 2004/000840 A2. Both methods use as reactants the amino alcohols of formula (II), the alkylating agents of formula (III) and the carbamoyl chlorides of formula (V) and proceed to the manufacture of the compounds of formula (I) through a two step reaction pathway. The yields obtained in each of the steps according to both methods as well as the global yield have been compiled in table 1.

**TABLE 1**

| | *Process of* WO 2004/000840 | | | *Process of the present invention* | | |
|---|---|---|---|---|---|---|
| | *Step 1* | *Step 2* | *Global* | *Step 1* | *Step 2* | *Global* |
| Example 1 | 18 | 79 | 14.2 | 100 | 46 | 46,0 |
| Example 2 | 22 | 48 | 10,6 | 96 | 42 | 40,3 |
| Example 3 | 22 | 62 | 13,6 | 97 | 70 | 67,9 |
| Example 4 | 29 | 72 | 20,9 | 100 | 32 | 32,0 |

As can be seen from the results shown in table 1 the process of the present invention provides a method for the manufacture of the compounds of formula (I) with better yields than those that could be obtained using methods of the prior art.

## Claims

1. A process for producing compounds of formula (I): 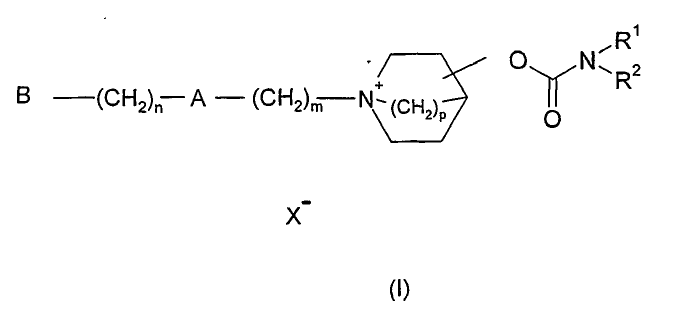 wherein
R¹ represents a group selected from phenyl, 2-furyl, 3-furyl, 2-thienyl, 3-thienyl, benzyl, furan-2-ylmethyl, furan-3-ylmethyl, thiophen-2-ylmethyl, thiophen-3-ylmethyl;
R² represents a group selected from optionally substituted lower alkyl, optionally substituted lower alkenyl, optionally substituted lower alkynyl, saturated or unsaturated cycloalkyl, saturated or unsaturated cycloalkylmethyl, phenyl, benzyl, phenethyl, furan-2-ylmethyl, furan-3-ylmethyl, thiophen-2-ylmethyl, thiophen-3-ylmethyl, pyridyl, and pyridylmethyl; wherein the carbocyclic moieties in the cycloalkyl, cycloalkylmethyl, phenyl, benzyl or phenethyl groups can be optionally bridged or fused to another saturated, unsaturated or aromatic carbocyclic moiety or to a cyclic moiety comprising carbon atoms and 1 or 2 oxygen atoms;
the cyclic groups present in R¹ and R² being optionally substituted by one, two or three substituents selected from halogen, straight or branched, optionally substituted lower alkyl, hydroxy, straight or branched, optionally substituted lower alkoxy, -SH, straight or branched optionally substituted lower alkylthio, nitro, cyano, -NR'R", -CO₂R', -C(O)-NR'R", -N(R"')C(O)-R', -N(R"')-C(O)NR'R", wherein R', R" and R'" each independently represents a hydrogen atom or a straight or branched, optionally substituted lower alkyl group or R' and R" together with the atom to which they are attached form a cyclic group;
p is 1 or 2 and the carbamate group is attached at positions 2, 3 or 4 of the azoniabicyclic ring,
m is an integer from 0 to 8;
n is an integer from 0 to 4;
A represents a group selected from -CH₂-, -CH=CR'-, -CR=CH-, -CR'R"-, -C(O)-, -O-, -S-, -S(O)-, -S(O)₂- and -NR'-, wherein R' and R" are as defined above;
B represents a hydrogen atom, or a group selected from straight or branched, optionally substituted lower alkyl, hydroxy, straight or branched, optionally substituted lower alkoxy, cyano, nitro, -CH=CR'R", -C(O)OR', -OC(O)R', -SC(O)R', -C(O)NR'R", -NR'C(O)OR", - NR"'C(O)NR'R", cycloalkyl, phenyl, naphthalenyl, 5,6,7,8-tetrahydronaphthalenyl, benzo[1,3]dioxolyl, heteroaryl or heterocyclyl; R', R" and R'" being as defined above; and wherein the cyclic groups present in group B are optionally substituted by one, two or three substituents selected from halogen, hydroxy, straight or branched, optionally substituted lower alkyl, phenyl, -OR', -SR', -NR'R", -NHCOR', -CONR'R", -CN, -NO₂ and - COOR'; R' and R" being as defined above;
X- represents a pharmaceutically acceptable anion of a mono or polyvalent acid;
including all individual stereoisomers of formulae (I) and mixtures thereof;
which process comprises the steps of:
a) Reacting a compound of formula (II) 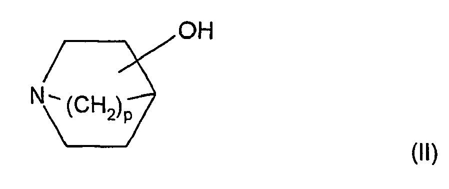 wherein p is as hereinabove defined with a compound of formula (III)
B -(CH₂)ₙ-A-(CH₂)ₘ-W (III)
wherein A, B, n and m are as hereinabove defined and wherein W is a leaving group
to yield a compound of formula (IV) 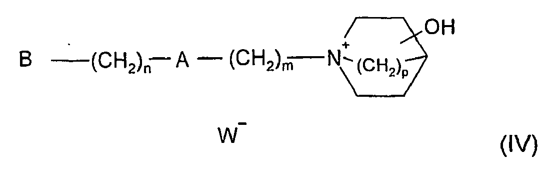
b) Subsequently reacting the compound of formula (IV) with an acylating agent of formula (V) 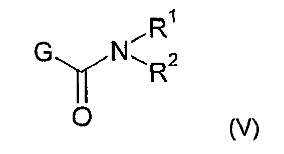 wherein R¹ and R² are as hereinabove defined and G represents a leaving group.
c) when W⁻ represents a group other than X⁻ exchanging the ion W⁻ in the product of step b) by the ion X- using standard methods of anion exchange
to yield the compounds of formulae (I), wherein a lower alkyl group contains 1 to 8, and a lower alkenyl or alkynyl group contains 2 to 8 carbon atoms.

2. A process according to claim 1 wherein the leaving group W is a halogen atom or a sulfonate ester group.

3. A process according to claim 1 or 2 wherein the leaving group G represents:
• an halogen atom
• a group of formula -OR^{IV}, wherein R^{IV} is a lower alkyl group, or an optionally substituted phenyl group
• an imidazol-1-yl group,
• an O-succinimidyl group.

4. A process according to claim 3 wherein the leaving group G represents a halogen atom.

5. A process according to claim 4 wherein the halogen atom is selected from the group consisting of chlorine and bromine.

6. A process according to claim 3 wherein the leaving group G represents a a group of formula -OR^{IV}.

7. A process according to claim 6 wherein the group R^{IV} is preferably selected from the group consisting of methyl, ethyl, phenyl or 4-nitrophenyl group

8. A process according to anyone of the preceding claims wherein the reaction of step a) is performed by reflux in tetrahydrofurane.

9. A process according to anyone of the preceding claims wherein the reaction of step b) is performed by addition of the compounds of formulae (IV) and (V) over a suspension of sodium hydride in dimethylformamide.

## Patentansprüche

1. Verfahren zur Herstellung von Verbindungen von Formel (I): 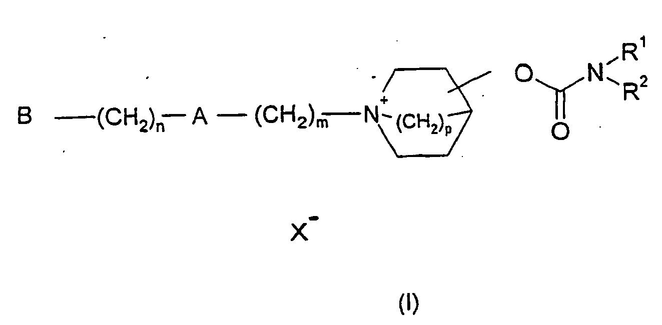 worin
R¹ für eine Gruppe steht, die ausgewählt ist aus Phenyl, 2-Furyl, 3-Furyl, 2-Thienyl, 3-Thienyl, Benzyl, Furan-2-ylmethyl, Furan-3-ylmethyl, Thiophen-2-ylmethyl, Thiophen-3-ylmethyl;
R² für eine Gruppe steht, die ausgewählt ist aus gegebenenfalls substituiertem Niederalkyl, gegebenenfalls substituiertem Niederalkenyl, gegebenenfalls substituiertem Niederalkinyl, gesättigtem oder ungesättigtem Cycloalkyl, gesättigtem oder ungesättigtem Cycloalkylmethyl, Phenyl, Benzyl, Phenethyl, Furan-2-ylmethyl, Furan-3-ylmethyl, Thiophen-2-ylmethyl, Thiophen-3-ylmethyl, Pyridyl und Pyridylmethyl; wobei die carbocyclischen Einheiten in den Cycloalkyl-, Cycloalkylmethyl-, Phenyl-, Benzyl- oder Phenethylgruppen gegebenenfalls verbrückt oder an eine weitere gesättigte, ungesättigte oder aromatische carbocyclische Einheit oder an eine cyclische Einheit, die Kohlenstoffatome und 1 oder 2 Sauerstoffatome umfasst, kondensiert sein können;
wobei die cyclischen Gruppen, die in R¹ und R² vorhanden sind, gegebenenfalls mit einem, zwei oder drei Substituenten substituiert sind, ausgewählt aus Halogen, geradem oder verzweigtem, gegebenenfalls substituiertem Niederalkyl, Hydroxy, geradem oder verzweigtem, gegebenenfalls substituiertem Niederalkoxy, -SH, geradem oder verzweigtem, gegebenenfalls substituiertem Niederalkylthio, Nitro, Cyano, -NR'R", -CO₂R', -C(O)-NR'R", -N(R"')C(O)-R', -N(R"')-C(O)NR'R", wobei R', R" und R"' jeweils unabhängig für ein Wasserstoffatom oder eine gerade oder verzweigte, gegebenenfalls substituierte Niederalkylgruppe stehen oder R' und R" zusammen mit dem Atom, an das sie gebunden sind, eine cyclische Gruppe bilden;
p 1 oder 2 ist und die Carbamatgruppe an den Positionen 2, 3 oder 4 des Azoniabicyclorings gebunden ist;
m eine ganze Zahl von 0 bis 8 ist;
n eine ganze Zahl von 0 bis 4 ist;
A für eine Gruppe steht, ausgewählt aus -CH₂-, -CH=CR'-, -CR'=CH-, -CR'R"-, -C(O)-, -O-, -S-, -S(O)-, -S(O)₂- und -NR'-, wobei R' und R" wie oben definiert sind;
B für ein Wasserstoffatom oder eine Gruppe steht, die ausgewählt ist aus geradem oder verzweigtem, gegebenenfalls substituiertem Niederalkyl, Hydroxy, geradem oder verzweigtem, gegebenenfalls substituiertem Niederalkoxy, Cyano, Nitro, -CH=CR'R", -C(O)OR', -OC(O)R', -SC(O)R', -C(O)NR'R", -NR'C(O)OR", -NR"'C(O)NR'R", Cycloalkyl, Phenyl, Naphthalinyl, 5,6,7,8-Tetrahydronaphthalinyl, Benzo[1,3]dioxolyl, Heteroaryl oder Heterocyclyl; wobei R', R" und R'" wie oben definiert sind; und wobei die cyclischen Gruppen, die in Gruppe B vorhanden sind, gegebenenfalls mit einem, zwei oder drei Substituenten substituiert sind, die ausgewählt sind aus Halogen, Hydroxy, geradem oder verzweigtem, gegebenenfalls substituiertem Niederalkyl, Phenyl, -OR', -SR', -NR'R", -NHCOR', -CONR'R", -CN, -NO₂ und -COOR'; wobei R' und R" wie oben definiert sind;
X- für ein pharmazeutisch verträgliches Anion einer ein- oder mehrwertigen Säure steht;
einschließlich aller einzelnen Stereoisomere der Formeln (I) und Mischungen davon;
wobei das Verfahren die Schritte umfasst:
a) Umsetzen einer Verbindung von Formel (II) 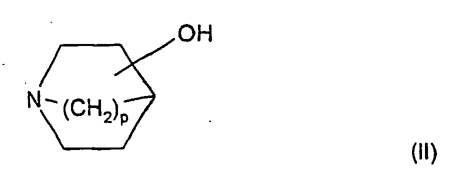 worin p ist, wie hierin zuvor definiert, mit einer Verbindung von Formel (III)
B -(CH₂)ₙ - A - (CH₂)ₘ -W (III)
worin A, B n und m sind, wie hierin zuvor definiert, und worin W eine Abgangsgruppe ist,
um eine Verbindung von Formel (IV) 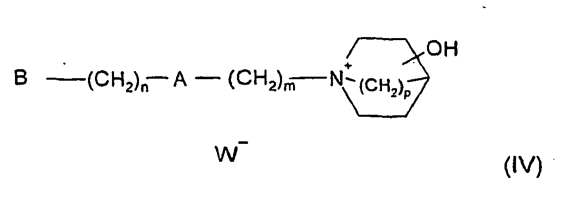 zu liefern,
b) anschließend Umsetzen der Verbindung von Formel (IV) mit einem Acylierungsmittel von Formel (V) 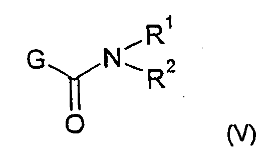 worin R¹ und R² sind, wie hierin zuvor definiert, und G für eine Abgangsgruppe steht,
c) wenn W⁻ für eine andere Gruppe als X⁻ steht, Austauschen des Ions W⁻ im Produkt von Schritt b) durch das Ion X⁻ unter Verwendung von Anionenaustauschstandardmethoden,
um die Verbindungen von Formel (I) zu liefern, wobei eine Niederalkylgruppe 1 bis 8 und eine Niederalkenyl oder -alkinylgruppe 2 bis 8 Kohlenstoffatome enthält.

2. Verfahren nach Anspruch 1, wobei die Abgangsgruppe W ein Halogenatom oder eine Sulfonatestergruppe ist.

3. Verfahren nach Anspruch 1 oder 2, wobei die Abgangsgruppe G für:
• ein Halogenatom
• eine Gruppe von Formel -OR^{IV}, worin R^{IV} eine Niederalkylgruppe oder eine gegebenenfalls substituierte Phenylgruppe ist
• eine Imidazol-1-ylgruppe
• eine O-Succinimidylgruppe
steht.

4. Verfahren nach Anspruch 3, wobei die Abgangsgruppe G für ein Halogenatom steht.

5. Verfahren nach Anspruch 4, wobei das Halogenatom ausgewählt ist aus der Gruppe, bestehend aus Chlor und Brom.

6. Verfahren nach Anspruch 3, wobei die Abgangsgruppe G für eine Gruppe von Formel -OR^{IV} steht.

7. Verfahren nach Anspruch 6, wobei die Gruppe R^{IV} vorzugsweise ausgewählt ist aus der Gruppe, bestehend aus Methyl-, Ethyl-, Phenyl- oder 4-Nitrophenylgruppe.

8. Verfahren nach einem der vorangehenden Ansprüche, wobei die Reaktion von Schritt a) durch Rückfluss in Tetrahydrofuran durchgeführt wird.

9. Verfahren nach einem der vorangehenden Ansprüche, wobei die Reaktion von Schritt b) durch Zugabe der Verbindungen der Formeln (IV) und (V) über eine Suspension von Natriumhydrid in Dimethylformamid durchgeführt wird.

## Revendications

1. Procédé de production de composés de formule (I) : 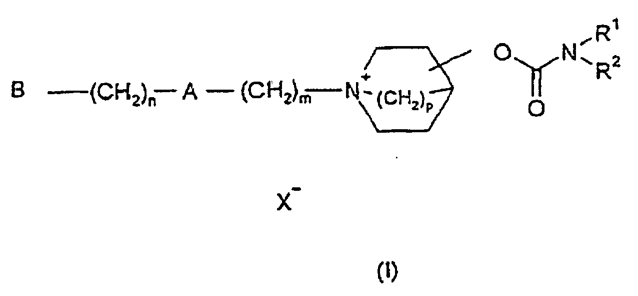 dans laquelle
R¹ représente un groupe choisi parmi un phényle, un 2-furyle, un 3-furyle, un 2-thiényle, un 3-thiényle, un benzyle, un furan-2-ylméthyle, un furan-3-ylméthyle, un thiophén-2-ylméthyle, un thiophén-3-ylméthyle ;
R² représente un groupe choisi parmi un alkyle inférieur facultativement substitué, un alcényle inférieur facultativement substitué, un alcynyle inférieur facultativement substitué, un cycloalkyle saturé ou insaturé, un cycloalkylméthyle saturé ou insaturé, un phényle, un benzyle, un phénéthyle, un furan-2-ylméthyle, un furan-3-ylméthyle, un thiophén-2-ylméthyle, un thiophén-3-ylméthyle, un pyridyle et un pyridylméthyle ; où les fragments carbocycliques dans les groupes cycloalkyle, cycloalkylméthyle, phényle, benzyle ou phénétyle peuvent être facultativement pontés ou fusionnés à un autre fragment carbocyclique saturé, insaturé ou aromatique ou à un fragment cyclique comprenant des atomes de carbone et 1 ou 2 atomes d'oxygène ;
les groupes cycliques présents dans R¹ et R² étant facultativement substitués avec un, deux ou trois substituants choisis parmi un halogène, un alkyle inférieur linéaire ou ramifié facultativement substitué, un hydroxy, un alcoxy inférieur linéaire ou ramifié facultativement substitué, -SH, un alkylthio inférieur linéaire ou ramifié facultativement substitué, un nitro, un cyano, -NR' R" , -CO₂R' -C (O) -NR' R" , -N (R"') C (O) - R' , -N(R"')-C(O)NR'R", où R', R" et R"' représentent chacun indépendamment un atome d'hydrogène ou un groupe alkyle inférieur linéaire ou ramifié facultativement substitué ou R' et R" ensemble avec l'atome auquel ils sont liés forment un groupe cyclique ;
p est 1 ou 2 et le groupe carbamate est fixé en position 2, 3 ou 4 du cycle azoniabicyclique,
m est un nombre entier de 0 à 8 ;
n est un nombre entier de 0 à 4 ;
A représente un groupe choisi parmi -CH₂-, -CH=CR'-, -CR'=CH-, -CR'R"-, -C (O) -, -O-, -S-, -S (O) -, -S(O)₂- et -NR'-, où R' et R" sont définis comme ci-dessus ;
B représente un atome d'hydrogène ou un groupe choisi parmi un alkyle inférieur linéaire ou ramifié facultativement substitué, un hydroxy, un alcoxy inférieur linéaire ou ramifié facultativement substitué, un cyano, un nitro, -CH=CR'R", -C(O)OR', -OC(O)R', -SC(O)R', -C(O)NR'R", -NR'C(O)OR", -NR'"C(O)NR'R", un cycloalkyle, un phényle, un naphtalényle, un 5,6,7,8-tétrahydronaphtalényle, un benzo[1,3]dioxolyle, un hétéroaryle ou un hétérocyclyle ; R', R" et R"' étant définis comme ci-dessus ; et où les groupes cycliques présents dans le groupe B sont facultativement substitués avec un, deux ou trois substituants choisis parmi un halogène, un hydroxy, un alkyle inférieur linéaire ou ramifié facultativement substitué, un phényle, -OR', -SR', -NR'R " , -NHCOR', - CONR'R", -CN, -NO₂ et -COOR' ; R' et R" étant définis comme ci-dessus ;
X⁻ représente un anion pharmaceutiquement acceptable d'un acide monovalent ou polyvalent ;
y compris tous les stéréoisomères individuels de formule (I) et leurs mélanges ;
ledit procédé comprenant les étapes suivantes :
a) la réaction d'un composé de formule (II) 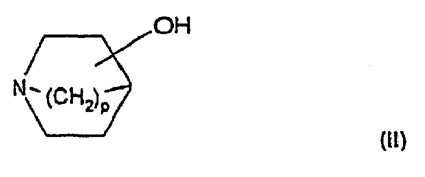 dans laquelle p est défini comme ci-dessus dans ce document, avec un composé de formule (III)
B -(CH₂)ₙ - A - (CH₂)ₘ -W (III)
dans laquelle A, B, n et m sont définis comme ci-dessus dans ce document et dans laquelle W est un groupe partant
pour donner un composé de formule (IV) 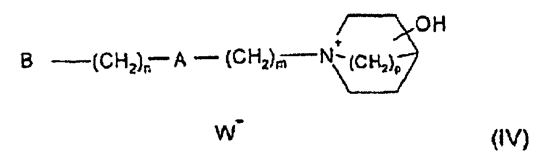
b) puis la réaction du composé de formule (IV) avec un agent d'acylation de formule (V) 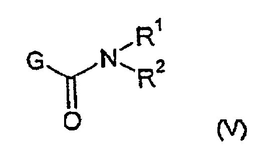 dans laquelle R¹ et R² sont définis comme ci-dessus dans ce document et G représente un groupe partant
c) quand W⁻ représente un groupe différent de X⁻, l'échange de l'ion W⁻ dans le produit de l'étape b) par l'ion X⁻ en utilisant des procédés standards d'échange d'anions
pour donner les composés de formule (I), dans lesquels un groupe alkyle inférieur contient 1 à 8, et un groupe alcényle ou alcynyle inférieur contient 2 à 8 atomes de carbone.

2. Procédé selon la revendications 1, dans lequel le groupe partant W est un atome d'halogène ou un groupe ester de sulfonate.

3. Procédé selon la revendication 1 ou 2, dans lequel le groupe partant G représente :
- un atome d'halogène
- un groupe de formule -OR^{IV}, dans laquelle R^{IV} est un groupe alkyle inférieur, ou un groupe phényle facultativement substitué
- un groupe imidazol-1-yle,
- un groupe O-succinimidyle.

4. Procédé selon la revendication 3, dans lequel le groupe partant G représente un atome d'halogène.

5. Procédé selon la revendication 4, dans lequel l'atome d'halogène est choisi dans le groupe constitué par le chlore et le brome.

6. Procédé selon la revendication 3, dans lequel le groupe partant G représente un groupe de formules -OR^{IV}.

7. Procédé selon la revendication 6, dans lequel le groupe R^{IV} est de préférence choisi dans le groupe constitué par un groupe méthyle, éthyle, phényle ou 4-nitrophényle.

8. Procédé selon l'une quelconque des revendications précédentes, dans lequel la réaction de l'étape a) est réalisée à reflux dans le tétrahydrofuranne.

9. Procédé selon l'une quelconque des revendications précédentes, dans lequel la réaction de l'étape b) est réalisée par l'ajout des composés de formules (IV) et (V) à une suspension d'hydrure de sodium dans du diméthylformamide.
